(19) Europäisches Patentamt
European Patent Office
Office européen des brevets

(11) **EP 1 857 100 A1**

(12) **EUROPEAN PATENT APPLICATION**

(43) Date of publication:
**21.11.2007 Bulletin 2007/47**

(51) Int Cl.:
*A61K 8/97* *(2006.01)*     *A61Q 19/08* *(2006.01)*

(21) Application number: **07107613.7**

(22) Date of filing: **07.05.2007**

(84) Designated Contracting States:
**AT BE BG CH CY CZ DE DK EE ES FI FR GB GR
HU IE IS IT LI LT LU LV MC MT NL PL PT RO SE
SI SK TR**
Designated Extension States:
**AL BA HR MK YU**

(30) Priority: **10.05.2006 IT PD20060182**

(71) Applicant: **Sala, Stefano**
**38100 Trento (IT)**

(72) Inventor: **Sala, Stefano**
**38100 Trento (IT)**

(74) Representative: **Modiano, Micaela Nadia et al**
**Dr. Modiano & Associati SpA**
**Via Meravigli 16**
**20123 Milano (IT)**

(54) **Cosmetic or pharmaceutical product for external use based on papaya**

(57) A cosmetic or pharmaceutical product for external use comprising papaya among its components, characterized in that the papaya is fermented.

**EP 1 857 100 A1**

**Description**

The present invention relates to a cosmetic or pharmaceutical product for external use based on papaya.

The properties of papaya have been known for many years.

Many of the properties of papaya derive from the presence of' papain, a proteolytic enzyme which is capable of degrading protein to peptides and of having a certain antiinflammatory and draining action.

For this reason, papaya is used as a traditional adjuvant for digestion problems (protein-rich meals), gastritis and gastroduodenitis and for weight loss regimens in people with diffuse and painful cellulitis.

Moreover, papaya is used for topical use for treating pediatric-age burns and for chronic skin ulcers in adults.

The enzymes contained in papaya, in addition to having a keratolytic action, in fact have exfoliating and antimicrobial properties on the skin, facilitating the elimination of necrotic tissue, the formation of granulation tissue and preventing bacterial superinfections in lesions.

The aim of the present invention is to provide a cosmetic or pharmaceutical product for external use which has exfoliating and smoothing properties and also allows to reduce the oxidation processes that lead to cell degeneration of the tissues to which it is applied.

Within this aim, an object of' the present invention is to provide a cosmetic or pharmaceutical product for external use which comprises papaya among its components and can be used in different types of application of a cosmetic product.

This aim and this and other objects, which will become better apparent hereinafter, are achieved by a cosmetic or pharmaceutical product for external use, characterized in that it comprises fermented papaya among its components.

In general, the invention consists in using fermented papaya to produce a cosmetic or pharmaceutical product for external application.

The physiological and health-related properties of food supplements based on papaya obtained after fermentation have in fact been studied for a few years.

In addition to the properties that are already typical of papaya before fermentation, described in the initial part of the present application, fermented papaya has excellent antioxidant properties; food supplements based on fermented papaya therefore allow to provide physiological benefits to many regions of' the body, helping to increase the general well-being of the individual.

As mentioned, the external, cosmetic use of papaya is limited to the non-fermented form, in order to achieve exfoliating, smoothing and lenitive actions on the skin.

Due to preconceptions, attention has never focused on the fact that fermented papaya could perform its beneficial antioxidant actions (in addition to exfoliating and smoothing actions) also in external use (skin, hair).

This preconception is confirmed by the fact that not a single cosmetic or pharmaceutical product for external use is currently known in the literature and commercially which comprises fermented papaya among its components.

The intuition on which the present invention is based, supported by subsequent testing and experimentation as set forth hereinafter, instead assumes that fermented papaya develops, in contact with the skin, an enhanced antioxidant action which allows to slow the oxidation processes that lead to skin aging.

Merely by way of example, the per se known type of technique that can be used to provide fermented papaya to be used as a component in cosmetic products is described hereafter.

Production is performed starting from the papaya fruit, which can be in various stages of ripeness: still green or with pulp which is already ripe and red.

It is possible to use equally the fruit in its entirety (peel, seeds, pulp), only the pulp with the seeds, the pulp with the peel, or the pulp alone.

The fruit that is used must be processed as puree and must have a sugar content of approximately 10° Brix.

Fermentation occurs by pouring the puree of the papaya fruit into a fermentation unit of' suitable capacity together with water in a quantity sufficient to render the preparation fluid enough to be mixed; then ferments, such as for example Saccharomyces Boulardii or other ferments such as lactobacilli, are added; fermentation has a variable duration until generation of $CO_2$ ceases.

At the end of fermentation, the fermented product is discharged and packaged in sterile drums; at this point it can be used as is or dehydrated.

Dehydration can occur for example by atomization or cryodesiccation.

The dehydrated product comprises over 30% carbohydrates, vitamin C and residual carotenoids in a variable percentage, proteolytic enzymes which are still significantly active, and live ferments if' it is cryodesiccated, or is substantially sterile if atomized.

As mentioned, fermented papaya has an enzymatic activity due substantially to the presence of the proteolytic enzymes, which perform an exfoliating and smoothing action on the skin, clearing it of the dead cells and gradually destroying them.

In addition to the enzymatic activity, there is an antioxidant activity which develops an antiaging action, reducing all the oxidation processes that lead to degeneration (aging) of the cells and tissue of the skin.

The antioxidant activity also leads to strengthening of the tissue, with a corresponding lenitive effect.

It has been verified that in a cosmetic or pharmaceutical product for external use, the quantitative interval of fermented papaya as a percentage on the total weight of the product is comprised between 0.01% and 30%.

Fermented papaya can be used in various forms of' application for topical use, such as for example creams, pastes, gels, lotions, mousses, sprays, et cetera.

The fields of application of' cosmetic or pharmaceutical products which use fermented papaya for external use are many: it is possible to use fermented papaya for example in:

- facial products with exfoliating, smoothing, lenitive, protective, anti-wrinkle, nutrient, antiaging and antioxidant activity;
- body products with exfoliating, smoothing, nutrient, lenitive, protective and antioxidant activity;
- hair products with strengthening, nutrient and loss-prevention activity;
- foot products with exfoliating, smoothing, nutrient, lenitive and protective activity;
- hand products with exfoliating, smoothing, nutrient, lenitive, protective and antioxidant activity;
- intimate hygiene products with lenitive and antioxidant activity;
- products for the mouth with lenitive and antioxidant activity;
- other products for external use.

In practice it has been found that the invention thus described achieves the intended aim and objects.

In particular, the present invention provides a cosmetic or pharmaceutical product for external use which has exfoliating, smoothing, lenitive properties and also allows, at the same time, to reduce oxidation processes which lead to degeneration of the cells of the tissues to which it is applied.

This has been achieved by using papaya of the fermented type in products for external use.

Experiments

Experiments were canted out under the supervision of the Department of' Molecular-Cellular Pharmacological and Physiologic Sciences from the University of Pavia, Italy, under the Research Protocol Numbers:

a) TV.010_2006/414;
b) FU.001S_FU.005S; and
c) FU.022S_2006/414

by the Dermatologic Cosmetic Centre for Wellness Farcoderm s.r.l. of Pavia, the contents of which are herewith incorporated by reference.

In summary, Protocol a) regards a test to evaluate if the tested raw materials (fermented papaya), at different percentages, possess any stimulating activity on collagen neosynthesis *in vitro.* For this purpose, their ability to increase the expressed level of collagen in human fibroblasts is investigated.

Cells were seeded in 96 wells plates, 2500 cells/well, for 24 h in DMEM (Dulbecco's Minimum Essential Medium) + 10% FCS (Fetal Calf Serum). Fresh medium was added, supplemented with only 5% FCS and with scalar dilutions of the tested products. The samples were dissolved in the medium. Untreated cells were used as negative controls. For each dilution, 2 replicate tests were performed and repeated twice. After 24 hours valued parameters had been determined on separate plates. Untreated cells were used as control.

Collagen synthesis was assessed using a commercially available kit (Sircol™, biodye science).

The assay was based on a Sirius Red dye capacity to interact with the side chain groups of' the basic amino acids present in collagen. The specific affinity of the dye for collagen, under the assay conditions was due to the elongated dye molecules becoming aligned parallel to the long, rigid structure of native collagens that have intact triple helix organisation.

Collagen neosynthesis was assessed in culture medium after treatment with the tested substance-material following kit instructions. A calibration curve was obtained with the collagen standard solution supplied with the kit.

**Collagen synthesis**

| mg/ml | [Collagen] µg/ml |
|---|---|
| 0.0 | 254.67 |
| 15 | 268.35 |
| 30 | 272.63 |
| 50 | 257.29 |

Increasing of Collagen Synthesis in human fibroblasts

It was so ascertained that the fermented papaya, according to the invention, is clearly effective in stimulating the collagen synthesis on cultured human fibroblasts, The following results were obtained, after 24 hours of incubation

- the 15 mg/ml papaya concentration determined an increase of 5.4% in the collagen released in the culture medium;
- the 30 mg/ml papaya concentration determined an increase of 7.0% in the collagen released in the culture medium;
- the 50 mg/ml papaya concentration determined an increase of 8.1% in the collagen released in the culture medium.

Under Protocol b), a test was carried out to check lenitive, emollient and moisturize efficacy of' a cosmetic papaya ingredient, according to the invention, used at 3 different concentrations. In particular, the experimental setup comprised the induction of an irritative reaction through the application of an epicutaneous patch filled with a disk paper saturated with Sodium Lauryl Sulfate (SLS, a strong irritant). The efficacy of the tested product to alleviate the experimentally-induced irritative reaction, was assessed. In particular, the data obtained for the emulsion with fermented papaya at 3 different concentrations were compared with the data obtained for the emulsion.

The study described was carried out, on human volunteers who gave their informed consent, following the Helsinky declaration principles for medical research.

Skin irritative reaction was induced through the application of an epicutaneous patch. Four "Finn chambers" of the patch were filled with a disk paper saturated with a 20% SLS aqueous solution. The patch was removed 4 hours after its application.

In this study the following instrumental parameters were monitored:

1. Trans epidermal water loss (TEWL) by means of Tewameter® method
2. Erythema index by means of Mexameter® method
3. Moisturization index by means of corneometer
4. Evaluation of skin profilometry by means of Visioscan® The parameters considered were: roughness and skin softness.

Instrumental readings were acquired at:

• T15: after 15 minutes from product application
• T30: after 30 minutes from product application
• T60: after 60 minutes from product application

Moreover, all the volunteers involved in the clinical test were treated with a lactic acid solution onto the nasolabial fold. 10" after the feeling of the burning/stinging pain (TO) the soothing treatment was applied on the right side of the face while on the other side we applied some drops of water only. One - three - five - ten and twenty minutes after, we registered the symptomatological and clinical (redness, desquamations, edema,...) differences according to the score resumed in table 1. Volunteers from 1 to 10 were treated with 3% of fermented papaya cream, while volunteers from 11 to 20 were treated with 5% of fermented papaya cream.

**TABLE 1**

| Stinging test (itching, stinging, pain) | |
|---|---|
| No pain | 0 |
| Light pain | 1 |
| Moderate pain | 2 |
| Strong pain | 3 |

The study was carried out in two phases:

1 - in this phase the irritative reaction was induced;
2 - in this phase the irritative reaction was instrumentally followed.

**PAPAYA 3%**

| TEWL VALUES | | | | | |
|---|---|---|---|---|---|
| VOLUNTEERS | BASIC VALUES | T0 | T15 | T30 | T60 |
| 01 MG | 18,4 | 18,2 | 13,3 | 7,3 | 7,7 |
| 02 LB | 8,0 | 10.3 | 10,6 | 6,3 | 7,6 |
| 03 BL | 11,2 | 9,1 | 9,1 | 5,3 | 6,5 |
| 04 RS | 12,6 | 7,3 | 14,8 | 6.6 | 3,3 |
| 05 RO | 9,4 | 22,5 | 11,8 | 6,4 | 5,1 |
| 06 SG | 12,7 | 19,8 | 12,4 | 10,2 | 8,9 |
| 07 AG | 16,6 | 27,0 | 23,6 | 8,1 | 5,6 |
| 08 RA | 14,8 | 28,5 | 12,6 | 9,0 | 6,0 |
| 09 PD | 13,9 | 18,8 | 10,5 | 8,4 | 7,0 |
| 10 LS | 11,8 | 17,0 | 11,3 | 9,3 | 7,9 |
| 11 BA | 7,5 | 17,0 | 10,4 | 9,7 | 8,9 |
| 12 BR | 12,3 | 15,9 | 17,4 | 14,3 | 15,5 |
| 13 CS | 9,5 | 9,3 | 14,6 | 10,3 | 8,3 |
| 14 MA | 13,7 | 10,7 | 38,3 | 37,4 | 33,7 |
| 15 DP | 14,1 | 26,9 | 22,6 | 21,7 | 18,0 |
| 16 VN | 15,2 | 19,4 | 22,3 | 1,4 | 17,7 |
| 17 CG | 13,6 | 17,8 | 17,5 | 16,6 | 12,9 |
| 18 AR | 15,0 | 12,5 | 17,4 | 16,5 | 12,8 |
| 19 EB | 16,7 | 10,7 | 38,3 | 37,4 | 33,7 |
| 20 PLM | 7,5 | 16,0 | 16,0 | 15,1 | 11,4 |
| Medium values | 12,73 | 16,74 | 17,24 | 13,87 | 11,93 |
| Dev SI | 3,12357336 | 6.243082 | 8,348741 | 9.39032 | 8,483102 |
| Test t basic vs | | | 0,006925 | 0,286 | 0,332017 |
| Test vs I0 | | 0,004282 | 0.419053 | 0,15069 | 0,039119 |

**PAPAYA 5%**

| TEWL VALUES | | | | | |
|---|---|---|---|---|---|
| VOLUNTEERS | BASIC VALUES | T0 | T15 | T30 | T60 |
| 01 MG | 19,7 | 13,6 | 18,4 | 11,8 | 10,6 |
| 02 LB | 8,0 | 9,5 | 12,8 | 9,0 | 8,6 |
| 03 BL | 12,6 | 6,4 | 6,2 | 4,8 | 5,6 |
| 04 RS | 10,2 | 9,7 | 9,5 | 6,8 | 5,6 |
| 05 RO | 8,8 | 20,1 | 10,0 | 9,3 | 6,4 |
| 06 SG | 9,8 | 25,6 | 16,4 | 12,1 | 11,1 |
| 07 AG | 16,6 | 21,0 | 21,0 | 8,0 | 6,4 |
| 08 RA | 15,7 | 33,7 | 14,1 | 11,9 | 8.3 |
| 09 PD | 11,2 | 19,2 | 11,7 | 8,4 | 9,7 |
| 10 LS | 14,4 | 14,7 | 10,5 | 9,4 | 8,0 |
| 11 BA | 15,7 | 20,3 | 10,5 | 10,5 | 9,7 |
| 12 BR | 12,3 | 15,0 | 14,7 | 16,9 | 15,0 |
| 13 CS | 9,5 | 11,2 | 14,8 | 12,1 | 10,3 |
| 14 MA | 15,0 | 13,6 | 22,0 | 18,5 | 17,3 |
| 15 DP | 16,6 | 27,9 | 23,6 | 25,8 | 17,7 |
| 16 VN | 16,8 | 17,4 | 23,8 | 20,2 | 19,0 |
| 17 CG | 14,6 | 19,5 | 21,6 | 19,3 | 18,1 |
| 18 AR | 18,2 | 7,4 | 25,2 | 26,7 | 21,5 |
| 19 EB | 15,0 | 17,9 | 22,0 | 18,5 | 17,3 |
| 20 PLM | 6,6 | 13,2 | 13,6 | 12,4 | 11,2 |
| Medium values | 13,37 | 16,85 | 16,12 | 13,62 | 11,87 |
| Dev ST | 3,6602092 | 6,93689 | 5,68123 | 6,14068 | 5,00821 |
| Test t basic vs | | | 0.00736 | 0,41828 | 0,08633 |
| Test t vs T0 | | 0,02132 | 0,34664 | 0,05491 | 0.00829 |

**PLACEBO EMULSION**

| TEWL VALUES | | | | | |
|---|---|---|---|---|---|
| VOLUNTEERS | BASIC VALUES | T0 | T15 | 130 | T60 |
| 01 MG | 19,7 | 17,4 | 26,7 | 27,0 | 22,8 |
| 02 LB | 8,0 | 7,6 | 15,0 | 15,3 | 11,1 |
| 03 BL | 7,0 | 6,8 | 14,0 | 14,5 | 10,3 |
| 04 RS | 9,4 | 6,8 | 16,4 | 16,9 | 12,7 |
| 05 RO | 10,7 | 14,6 | 22,6 | 1 4,5 | 15,3 |
| 06 SG | 11,9 | 22,7 | 26,8 | 27,3 | 19,2 |
| 07 AG | 12,3 | 18,1 | 13,6 | 13,9 | 9.7 |
| 08 RA | 13,8 | 12,6 | 14,1 | 13,7 | 9,5 |

(continued)

| TEWL VALUES | | | | | |
|---|---|---|---|---|---|
| VOLUNTEERS | BASIC VALUES | T0 | T15 | 130 | T60 |
| 09 PD | 9,7 | 28,7 | 13,6 | 10,4 | 6,2 |
| 10 LS | 16,6 | 30,2 | 23,6 | 25,8 | 14,7 |
| 11 BA | 16,8 | 17,4 | 23,8 | 20,2 | 16,0 |
| 12 BR | 14,6 | 19,5 | 21,6 | 19,3 | 15,1 |
| 13 CS | 8,7 | 7,4 | 25,2 | 26,7 | 18,5 |
| 14 MA | 15,0 | 30,2 | 22,0 | 18,5 | 14,3 |
| 15 DP | 16,6 | 30,2 | 23,6 | 25,8 | 14,7 |
| 16 VN | 16,8 | 17,4 | 23,8 | 20,2 | 16,0 |
| 17 CG | 14,6 | 19,5 | 21,6 | 19,3 | 15,1 |
| 18 AR | 18,2 | 7,4 | 25,2 | 26,7 | 18,5 |
| 19 EB | 15,0 | 30,2 | 22,0 | 18,5 | 14,3 |
| 20 PLM | 6,6 | 13,2 | 13,6 | 12,4 | 8,2 |
| Medium values | 13,10 | 17,90 | 20,44 | 19,35 | 14,11 |
| Dev ST | 3,9174374 | 8,53732 | 4,85402 | 5,50287 | 4,07184 |
| Test t basic vs | | | 1,8E-08 | 2,9E-06 | 0,1101 |
| Test t vs I0 | | 0,0058 | 0,10283 | 0,25184 | 0,0448 |

Medium values for TEWL were obtained for the compositions of fermented papaya cream that are shown in the following table:

The medium Erithema index values found were:

Erithema index

The medium Moisturization values measured were:

Moisturization values

The medium skin roughness values were:

Ser:skin roughness

The medium skin scaliness values measured were:

SESc:skin scaliness

In all of the above tests a significant overall improvement of the skin measured parameters was ascertained after 15-30 minutes from the application of the fermented papaya cream with the concentrations, by weight, mentioned.
Under Protocol c) a study was carried out aimed to evaluate the Keratolytic activity of a raw, fermented papaya material for cosmetic use.

9

The study was carried out according to a double-blinded protocol with randomisation of the application sites.

The randomisation scheme of the application sites of the product and of the positive control was saved by the study director.

Under the test, irritating products were applied to the skin of the volunteers by means of an occlusive chamber (Finn chamber, Ø 7.0 mm) containing a disc of absorbing paper wetted with a known quantity (20 μl) of product.

The exposure time to the irritating product (Salicylic acid at 1.5, 3.0 and 5.0% (w/v) concentration) was 3 and 6 hours.

The following parameters were evaluated at each experimental time:

- desquamation index
- clinical analysis of the adverse skin reactions.

The data obtained were reported both in absolute value and as variation percentage.

The data obtained for the substance tested were compared with those obtained with the positive control.

The irritating product was provided as solutions.

The solutions of salicylic acid were prepared as follows:

SOLUTION 1.5%: 1.5 g of salycilic acid were dissolved in the smallest possible volume of ethanol and then in distilled water (the amount that is needed at 100 ml);

SOLUTION 3.0%: 3.0 g of' salycilic acid were dissolved in the smallest possible volume of ethanol and then in distilled water (the amount that is needed at 100 ml);

SOLUTION 5.0%: 5.0 g of salycilic acid were dissolved in the smallest possible volume of ethanol and then in distilled water (the amount that is needed at 100 ml).

Further a fermented papaya powder of a 100% pureness was obtained and an active product made as follows:

SOLUTION 1.5%: 1.5 g of fermented papaya in 100 ml of distilled water;

SOLUTION 3.0%: 3.0 g of fermented papaya in 100 ml of distilled water;

SOLUTION 5.0%: 5.0 g of fermented papaya in 100 ml of distilled water.

The application site of the irritating products selected for study purposes was the volar surface of the forearm.

The products were applied on the skin of the volunteers for 3 and 6 hours.

After 15 minutes of each patch removal the following parameters were evaluated:

☐ a) skin redness - The skin reactions were classified according to the clinical scores reported in table 1.

**TABLE I -** *Evaluation scale*

| No reactions | 0 |
|---|---|
| Weakly positive reaction (generally, characterized by light erythema and/or dryness that might diffuse even beyond the treatment site) | 1 |
| Moderately positive reaction (generally, characterized by light erythema and/or dryness that might diffuse even beyond the treatment site) | 2 |
| Strongly positive reaction (strong erythema, often diffuse, with oedema and/or eschar formation) | 3 |

☐ b) desquamation index on the images acquired with Corneofix® tape and Visioscan® (Courage + Khazaka electronic GmbH). Desquamation index was calculated as:

$$DI = [2A + (T1 \cdot (1 - 1) + T2 \cdot (2 - 1) + ... + T_n \cdot (n - 1)] / (n + 1)$$

where:

A: percentage area covered by corneocytes
$T_n$: % of corneocytes related to their thickness
n: number of thickness levels.

The comparative results as regards the desquamation test obtained between the effects of the irritating and fermented papaya products are as follows.

The fermented papaya was noted to determine a significant increase of desquamation index both after 3.0 and 6.0 hours of' application. The statistical analysis evidenced that the maximum keratolytic action was reached after 3 hours and at 3.0% concentration.

The Salicylic acid was noted to determine an increase of desquamation index both after 3.0 and 6.0 hours of' application. The statistical analysis evidenced that the maximum keratolytic action was reached after 3 hours and at 3.0% concentration.

| Variation | FERMENTED PAPAYA | | | SALICYLIC ACID | | |
|---|---|---|---|---|---|---|
| | | | | | | |
| | 1.5% | 3.0% | 5.0% | 1.5% | 3.0% | 5.0% |
| 3 h | $2.69\pm0.39$ | $3.67\pm0.44$ | $3.77\pm0.43$ | $2.58\pm0.24$ | $3.12\pm0.24$ | $3.25\pm0.26$ |
| 6 h | $2.83\pm0.43$ | $3.60\pm0.52$ | $3.73\pm0.47$ | $2.71\pm0.36$ | $3.24\pm0.38$ | $3.59\pm0.43$ |

*The variation was calculated as:

$$\frac{I.D.\ [X]_0}{I.D.\ [X]_x}$$

Where

I.D. $[X]_0$: Basal desquamation index;
I.D. $[X]_x$: Desquamation index at the x concentration (1.5, 3.0 and 5.0%) of fermented papaya or salicylic acid.

The results obtained for fermented papaya and for salicylic acid were noted to be comparable.

In both cases:

1. the maximum keratolytic efficacy was obtained after 3 hours of application;
2. the maximum keratolytic efficacy was obtained at a 3.0% concentration.

In addition, the medium skin irritation index after 6 hours from application was comparable for the two products (the irritating and papaya products).

Advantageously, a very good skin tolerance, with no adverse or dangerous action for the fermented papaya product was generally ascertained.

Products with lower concentrations of fermented papaya, as low as 0,01% by weight, would therefore allow noteworthy benefits.

Products with higher concentrations of fermented papaya, of up to 30% by weight would also bring in a still better moisturing of the skin and lower roughness thereof without increasing skin risks. Moreover with such higher concentration, a still more efficient while not harmful skin desquamation may be achieved.

The invention thus conceived is susceptible of numerous modifications and variations, all of which are within the scope of the appended claims; all the details may further be replaced with other technically equivalent elements.

In practice, the components used in combination with fermented papaya, so long as they are compatible with the specific use, as well as their quantity, may be any according to requirements and according to the state of the art.

The disclosures in Italian Patent Application No. PD2006A000182 from which this application claims priority are incorporated herein by reference.

**Claims**

1. A cosmetic or pharmaceutical product for external use, comprising papaya among its components, **characterized in that** said papaya is fermented.

2. The cosmetic or pharmaceutical product for external use according to claim 1, **characterized in that** said fermented papaya is present in a quantity comprised between 0.01% and 30% by weight of the total weight of the product.

**3.** Use of fermented papaya to provide a cosmetic or pharmaceutical product for external application.

EP 1 857 100 A1

**European Patent Office**

**EUROPEAN SEARCH REPORT**

Application Number

EP 07 10 7613

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/271751 A1 (PERRIER ERIC [FR] ET AL) 8 December 2005 (2005-12-08) * claim 42; examples 19,34-38 * | 1-3 | INV. A61K8/97 A61Q19/08 |
| X | DATABASE WPI Week 200430 Derwent Publications Ltd., London, GB; AN 2004-323448 XP002446141 & JP 2004 123585 A (SAIDO KK) 22 April 2004 (2004-04-22) * abstract * | 1-3 | |
| X | DATABASE WPI Week 200626 Derwent Publications Ltd., London, GB; AN 2006-244777 XP002446142 & JP 2006 075086 A (TOYO SHINYAKU KK) 23 March 2006 (2006-03-23) * abstract * | 1-3 | |
| X | DATABASE WPI Week 198528 Derwent Publications Ltd., London, GB; AN 1985-169428 XP002446143 & JP 60 100506 A (ISHII T) 4 June 1985 (1985-06-04) * abstract * | 1-3 | TECHNICAL FIELDS SEARCHED (IPC) A61K A61Q |
| X | WO 03/059368 A (COGNIS FRANCE SA [FR]; MOUSSOU PHILIPPE [FR]; DANOUX LOUIS [FR]; PAULY) 24 July 2003 (2003-07-24) * page 3, line 19; claims 1-3,5,28 * | 1-3 | |

The present search report has been drawn up for all claims

| Place of search | Date of completion of the search | Examiner |
|---|---|---|
| Munich | 9 August 2007 | Mitchell, Gemma |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

13

## ANNEX TO THE EUROPEAN SEARCH REPORT
## ON EUROPEAN PATENT APPLICATION NO.

EP 07 10 7613

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2007

| Patent document cited in search report | | Publication date | Patent family member(s) | Publication date |
|---|---|---|---|---|
| US 2005271751 | A1 | 08-12-2005 | DE 102004045187 A1<br>FR 2871061 A1<br>GB 2414669 A<br>JP 2005343884 A<br>KR 20050115817 A | 29-12-2005<br>09-12-2005<br>07-12-2005<br>15-12-2005<br>08-12-2005 |
| JP 2004123585 | A | 22-04-2004 | NONE | |
| JP 2006075086 | A | 23-03-2006 | NONE | |
| JP 60100506 | A | 04-06-1985 | NONE | |
| WO 03059368 | A | 24-07-2003 | AU 2003205571 A1<br>EP 1461059 A1<br>FR 2834718 A1<br>JP 2005521649 T<br>US 2005089499 A1 | 30-07-2003<br>29-09-2004<br>18-07-2003<br>21-07-2005<br>28-04-2005 |

EPO FORM P0459

For more details about this annex : see Official Journal of the European Patent Office, No. 12/82

**EP 1 857 100 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IT PD20060182 A